(19) Europäisches Patentamt
European Patent Office
Office européen des brevets

(11) EP 4 322 942 B1

(12) EUROPEAN PATENT SPECIFICATION

(45) Date of publication and mention of the grant of the patent:
**30.04.2025 Bulletin 2025/18**

(21) Application number: **22722256.9**

(22) Date of filing: **12.04.2022**

(51) International Patent Classification (IPC):
***A61K 31/4015*** (2006.01) ***A61K 31/436*** (2006.01)
***A61P 35/04*** (2006.01)

(52) Cooperative Patent Classification (CPC):
(C-Sets available)
**A61P 35/04; A61K 31/4015; A61K 31/436** (Cont.)

(86) International application number:
**PCT/EP2022/059704**

(87) International publication number:
**WO 2022/218958 (20.10.2022 Gazette 2022/42)**

# (54) COMBINATION COMPRISING EVEROLIMUS AND AMCENESTRANT

KOMBINATION MIT EVEROLIMUS UND AMCENESTRANT

COMBINAISON COMPRENANT DE L'ÉVÉROLIMUS ET DE L'AMCENESTRANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2021 EP 21315063**

(43) Date of publication of application:
**21.02.2024 Bulletin 2024/08**

(73) Proprietor: **Sanofi**
**75017 Paris (FR)**

(72) Inventors:
- **BOUABOULA, Monsif**
  **Cambridge, Massachusetts 02142 (US)**
- **SUN, Fangxian**
  **Cambridge, Massachusetts 02142 (US)**

(74) Representative: **Cabinet Nony**
**11 rue Saint-Georges**
**75009 Paris (FR)**

(56) References cited:
**WO-A1-2017/140669** **WO-A1-2021/178846**
**US-A1- 2016 184 311**

- **ANONYMUS: "History of Changes for Study: NCT03284957", 25 March 2021 (2021-03-25), XP093025690, Retrieved from the Internet <URL:https://clinicaltrials.gov/study/NCT03284957?tab=history&a=47#version-content-panel> [retrieved on 20230221]**
- **ANONYMOUS: "Phase 1 / 2 Study of Amcenestrant (SAR439859) Single Agent and in Combination With Other Anti-cancer Therapies in Postmenopausal Women With Estrogen Receptor Positive Advanced Breast Cancer", 15 September 2017 (2017-09-15), XP055919472, Retrieved from the Internet <URL:https://www.clinicaltrials.gov/ct2/show/NCT03284957> [retrieved on 20220510]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/4015, A61K 2300/00;**
**A61K 31/436, A61K 2300/00**

## Description

Herein are provided a combination of everolimus and amcenestrant, a pharmaceutical composition containing such combination, and the therapeutic uses of such combination and pharmaceutical composition for the treatment of breast cancer.

The estrogen receptor $\alpha$ (ESR1) is expressed in the majority of breast tumors, enabling them to respond to the mitogenic actions of estrogens.

Amcenestrant, INN name for the compound 6-(2,4-dichlorophenyl)-5-[4-[(3S)-1-(3-fluoropropyl)pyrrolidin-3-yl]oxyphenyl]-8,9-dihydro-7H-benzo[7]annulene-2-carboxylic acid (also known by its laboratory code SAR439859), is a selective estrogen receptor degrader (SERD) which is an estrogen receptor antagonist and accelerates the proteasomal degradation of the estrogen receptor. This compound is disclosed in the patent application WO 2017/140669:

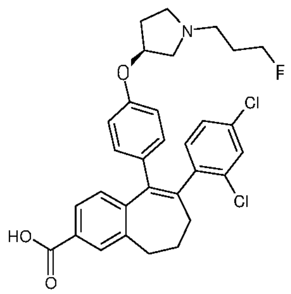

Everolimus (INN name) is an inhibitor of mammalian target of rapamycin (mTOR). It has the following formula:

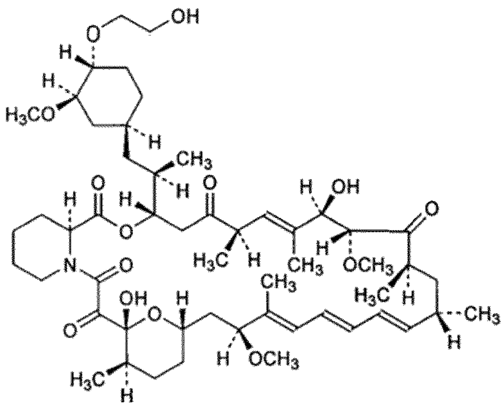

Everolimus is a marketed drug, with AFINITOR® as one of its tradenames. It is indicated in several oncology settings. In breast cancer, everolimus is indicated for the treatment of postmenopausal women with advanced hormone receptor (HR)-positive, human epidermal growth factor receptor 2 (HER2)-negative breast cancer in combination with exemestane after failure of treatment with letrozole or anastrozole (FDA label).

There is always a need to find new antitumoral treatments. Now, it is shown herein that a combination of amcenestrant with everolimus demonstrates significant anti-tumor efficacy, and induces tumor regression, with a synergistic effect compared to each of the active ingredient alone.

Herein is provided a combination comprising amcenestrant and everolimus for use in the treatment of breast cancer. The present invention is defined by the independent claims. The dependent claims depict other embodiments of the invention. Any embodiment not falling under the scope of the appended claims does not form part of the invention.

In the combination provided herein, amcenestrant may exist not only in the form of a zwitterion (i.e., a globally neutral molecule having an acid group and a basic group), but also in the form of addition salts with acids or bases. Such addition salts may be used in the above combination. Hence, herein is provided a combination comprising amcenestrant, or a pharmaceutically acceptable salt thereof, and everolimus.

In an embodiment, the combination of amcenestrant, or a pharmaceutically acceptable salt thereof, with everolimus shows therapeutic synergy. A combination demonstrates therapeutic synergy if its therapeutic effect is superior compared to the cumulative effect of either active agent of the combination alone.

In another embodiment, amcenestrant, or a pharmaceutically acceptable salt thereof, and everolimus are administered orally.

Provided herein is also a combination of amcenestrant, or a pharmaceutically acceptable salt thereof, and

everolimus for its use as a medicament.

Provided herein is also a pharmaceutical composition comprising amcenestrant, or a pharmaceutically acceptable salt thereof, and everolimus, as well as at least one pharmaceutically acceptable excipient.

The excipients are selected from the customary excipients which are known to a person skilled in the art. More particularly, the excipients are selected from those useful for oral administration in whatever form (liquid solution, dispersion or suspension, tablets, capsules, or the like).

In another embodiment, amcenestrant, or a pharmaceutically acceptable salt thereof, and everolimus may be administered simultaneously, separately, or spaced out over a period of time (sequential administration). Therefore, the combination and pharmaceutical composition provided herein are not exclusively limited to the ones which are obtained by physical association of the constituents in a single unit dosage, but also to those which allow a separate administration, which can be simultaneous or sequential (also called "spaced out" or "spread out") over a period of time.

Herein is also provided a pharmaceutical kit which comprises:

(i) a first pharmaceutical composition comprising amcenestrant, or a pharmaceutically acceptable salt thereof, and at least one pharmaceutically acceptable excipient;
(ii) a second pharmaceutical composition comprising everolimus, and at least one pharmaceutically acceptable excipient;

wherein the first pharmaceutical composition and the second pharmaceutical composition are in separate compartments and are intended to be independently administered, each administration with regards to the other one being simultaneous or spaced out (sequential) over time, for use in the treatment of breast cancer.

In the combinations, pharmaceutical compositions and pharmaceutical kit described above, amcenestrant, or a pharmaceutically acceptable salt thereof, and everolimus are advantageously present at effective doses, adapted considering the treated pathology and the condition of the patient to which the combination is administered. In particular, for everolimus the recommended dose for adult patients is 10 mg once daily, taken orally.

Herein is also provided a combination comprising amcenestrant, or a pharmaceutically acceptable salt thereof, and everolimus, as well as a pharmaceutical composition and kit as described above, for use in the treatment of cancer.

Herein is also provided amcenestrant or a pharmaceutically acceptable salt thereof for use in the treatment of cancer by co-administration with everolimus.

Herein is also provided everolimus for use in the treatment of cancer by co-administration with amcenestrant or a pharmaceutically acceptable salt thereof.

Co-administration is understood herein as an administration of the active ingredients to a patient in need thereof, which is separated, simultaneous or spaced out (sequential) over time, with respect to each of the active ingredients.

In some embodiments, amcenestrant, or a pharmaceutically acceptable salt thereof, and everolimus are administered in a therapeutically effective amount. A "therapeutically effective amount" means the amount of an active ingredient or combination of active ingredients that, when administered to a patient for treating a disease, is sufficient to affect such treatment for the disease. The "therapeutically effective amount" will vary depending on the disease and its severity and the age, weight, etc., of the subject to be treated.

In some embodiments, amcenestrant, or a pharmaceutically acceptable salt thereof, and everolimus are administered in an amount to show therapeutic synergy.

In another embodiment, the cancer is a hormone dependent cancer.

In another embodiment, the cancer is an estrogen receptor dependent cancer, particularly the cancer is an estrogen receptor $\alpha$ dependent cancer.

The references to methods of treatment by therapy in this description are to be interpreted as references to compounds, pharmaceutical compositions and medicaments of the present invention for use in those methods.

Herein is also provided a method of treating the pathological conditions indicated above, particularly breast cancer, comprising administering to a subject in need thereof a therapeutically effective amount of amcenestrant, or a pharmaceutically acceptable salt thereof, and a therapeutically effective amount of everolimus.

Herein is also provided a method of treating the pathological conditions indicated above, particularly breast cancer, comprising administering to a subject in need thereof a pharmaceutical composition or a pharmaceutical kit as described above.

Herein is also provided a method of treating the pathological conditions indicated above, particularly breast cancer, comprising administering to a subject in need thereof a combination as described above.

Herein is also provided a method of treating the pathological conditions indicated above, particularly breast cancer, comprising co-administering to a subject in need thereof amcenestrant or a pharmaceutically acceptable salt thereof and everolimus. In said method, amcenestrant, or a pharmaceutically acceptable salt thereof, is administered with everolimus either separately, simultaneously or spaced out over time.

Herein is also provided a method of treating the pathological conditions indicated above, particularly breast

cancer, comprising co-administering to a subject in need thereof everolimus and amcenestrant, or a pharmaceutically acceptable salt thereof. In said method, everolimus is administered with amcenestrant, or a pharmaceutically acceptable salt thereof, either separately, simultaneously or spaced out over time.

Herein is also provided a method of treating cancer comprising administering to a patient in need thereof a therapeutically effective amount of amcenestrant or a pharmaceutically acceptable salt thereof in combination with a therapeutically effective amount of everolimus.

Herein is also provided a method of treating cancer in a patient who is on therapy with compound amcenestrant, or a pharmaceutically acceptable salt thereof, comprising administering to said patient an effective amount of everolimus.

Herein is also provided a method of treating cancer in a patient on stable treatment with compound amcenestrant, or a pharmaceutically acceptable salt thereof, comprising administering to said patient a therapeutically effective amount of everolimus.

Herein is also provided a method of treating cancer comprising administering to a patient in need thereof a therapeutically effective amount of compound everolimus, wherein said patient is also on therapy with amcenestrant or a pharmaceutically acceptable salt thereof.

In an embodiment of the methods described above, the subject is a mammal. In another embodiment, the subject is a human.

Herein is also provided a combination comprising amcenestrant, or a pharmaceutically acceptable salt thereof, and everolimus for the manufacture of a medicament useful in treating the pathological conditions indicated above, particularly breast cancer.

Herein is also provided the use of amcenestrant, or a pharmaceutically acceptable salt thereof, in the manufacture of a medicament useful in treating the pathological conditions indicated above, particularly breast cancer, by co-administration with everolimus.

Herein is also provided the use of everolimus in the manufacture of a medicament useful in treating the pathological conditions indicated above, particularly breast cancer, by co-administration with amcenestrant or a pharmaceutically acceptable salt thereof.

Herein is also provided an article of manufacture, a packaging, or an administration unit, comprising:

- a packaging material;
- the above defined combination, pharmaceutical composition, or pharmaceutical kit; and
- a label or package insert contained within said packaging material, indicating that said combination, pharmaceutical composition or pharmaceutical kit is administered to a patient for the treatment of cancer.

The examples below show the pharmacological results obtained with amcenestrant, everolimus and their combination against a breast cancer cell line xenograft in NOD/SCID (nonobese diabetic/severe combined immunodeficiency) mice.

**Evaluation of the efficacy of amcenestrant combined with everolimus against a subcutaneous breast cancer cell line xenograft in female NOD/SCID mice**

In the present study, the anti-tumor efficacy of amcenestrant combined with everolimus was investigated after 28 days treatment against an orthotopic MCF7 human breast cancer cell line xenograft in female NOD/SCID mice.

The treated groups included amcenestrant at 10 mg/kg alone, everolimus at 10 mg/kg alone, and the combination of amcenestrant and everolimus at the same dose and regime.

For 28 days, amcenestrant was orally dosed twice a day (BID), and everolimus was orally dosed twice a week (BIW). Anti-tumor efficacy was evaluated by tumor volume measurement.

**1: Experimental procedure**

1-1: Animals, cell line, compounds

Female NOD/SCID mice were obtained from GemPharmatech Co.,Ltd (Nanjing University National Resource Center for Mutant Mice, Model Animal Research Center) (Nanjing, CHINA). Animals were allowed to acclimate for at least four days before the study enrollment. Mice were 7 to 8 weeks old and weighed between 22.5 and 28.4 grams at the beginning of the treatments. These animals were housed under conditions outlined in the guidelines approved by the Institutional Animal Care and Use Committee (IACUC) of CrownBio following the guidance of the Association for Assessment and Accreditation of Laboratory Animal Care (AAALAC).

MCF7 cells were obtained from the American Type Culture Collection (ATCC® HTB-22™). The MCF7 tumor cells were maintained in vitro with MEM medium supplemented with 10% fetal bovine serum, 0.01mM non-essential amino

acid, 0.01 mg/ml bovine insulin, and 2 mM L-glutamine at 37°C in an atmosphere of 5% CO2 in air. The cells in exponential growth phase were harvested and quantitated by cell counter before tumor inoculation.

The mouse was subcutaneously implanted with estrogen pellets (0.18 mg/pellet, 17β-estradiol, Innovative Research of America, Sarasota, Florida, USA), hereafter referred to as "E2 supplement", at the right flank one day before the tumor inoculation. Each mouse was inoculated with MCF-7 tumor cells (2 x 10e7) in 0.25 ml of PBS/Matrigel (1:1) mixture at the right mammary fat pad for tumor development.

Everolimus (Manufacturer: Selleck; Lot number: S1120) was formulated in 30% propylene glycol (dissolve first): 5% Tween 80: 65% dd$H_2O$ (double distilled water).

Dose volume for amcenestrant and everolimus for oral administration: 10 ml/kg.

Doses: amcenestrant at 10 mg/kg and everolimus at 10 mg/kg in the above volume.

1-2: Study design, end points

The animals required for experiment (plus extra) were pooled and implanted with MCF7 cells. On day 0 (12 days post implantation), the mice were pooled and randomly distributed to the treatment and control groups (10 mice per group), where median tumor volumes for each group was 119 $mm^3$. Treatments of amcenestrant and everolimus were initiated on day 1. For 28 days, amcenestrant was orally administered at 10 mg/kg BID (8 hours apart), and everolimus was orally administered at 10 mg/kg BIW. Animal body weight was assessed daily.

The dosages are expressed in mg/kg and based on daily body weight per animal. Vehicle treated animals were used as controls. Mice were checked daily for adverse clinical reactions. Individual mice were weighed daily until the end of the experiment. Mice were euthanized when morbid or weight loss ≥20% was observed. Tumors were measured with a caliper twice weekly until final sacrifice. When a tumor size reached approximately 3000 $mm^3$ or when there were animal health issues (40% area of a tumor ulcerated), animals were euthanized, and date of death recorded. Solid tumor volumes were estimated from two-dimensional tumor measurements and calculated according to the following equation:

$$Tumor\ volume\ \left(mm^3\right) = \frac{length\ (mm) \times width^2\ (mm^2)}{2}$$

Toxicity end points:

A dosage producing either 15% body weight loss during 3 consecutive days for an individual mouse, 20% body weight loss during 1 day, or 10% or more drug related deaths were considered an excessively toxic dosage, unless under certain circumstances bodyweight loss or animal death could be considered non-drug related. Examples include E2 supplement related body weight loss and urine scalding, animal handling issues such as misgavage, tumor model related issues such as tumor induced cachexia leading to body weight loss that can be observed in control, or vehicle treated groups and excessive tumor ulceration. Mice that had non-drug related death or significant bodyweight loss were not considered toxic and were excluded from statistical analysis. Animal body weight included the tumor weight.

Efficacy end points:

The primary efficacy end points include tumor volume changes from baseline summarized by the ratio of medians of tumor volume changes from baseline between the treated and control groups (ΔT/ΔC). Changes in tumor volume for each treated (T) and control (C) group were calculated for each animal on each day by subtracting the tumor volume on the day of first treatment (staging day) from the tumor volume on the specified observation day. The median ΔT was calculated for the treated group and the median ΔC was calculated for the control group. The ratio ΔT/ΔC was calculated and expressed as percentage:

$$\Delta T\ /\ \Delta C = \left(\frac{Median\, deltaT}{Median\, deltaC}\right) \times 100$$

ΔT/ΔC ≤ 40% is considered as therapeutically active, ΔT/ΔC = 0% is considered as tumor stasis, and ΔT/ΔC < 0% is considered as tumor regression (very active). ΔT/ΔC > 40% is considered as therapeutically inactive.

Percent tumor regression is defined as % (percentage) of tumor volume decrease in the treated group on a specified observation day compared to its volume when the study was initiated. At a specific time point (t) and for each animal, the regression percentage was calculated using the following formula:

$$\% \ regression \ (at \ t) = \left( \frac{volume_{t0} - volume_{t}}{volume_{t0}} \right) \times 100$$

The median percent regression for a group on a given day was then calculated by taking the median of individual % regression values calculated for each animal in the group. The day of calculation was determined by the day when ΔT/ΔC is calculated, except if median percent regression was not representative of the activity of the group. In this case, the day was determined by the first day when the median percent regression was maximal.

1-3: Statistical analysis

A two-way analysis of variance (ANOVA) with factors treatment and day (repeated) was performed on tumor volume changes from baseline. It was followed by contrast analyses with Bonferroni-Holm correction for multiplicity to compare all treated groups to the control group and to compare the combination *versus* each single agent at the dose involved in the combination at each day from day 4 to 28.

In the figures, the medians and Median Absolute Deviation (MAD) of each group are represented for each day of measurement.

In the tables, the medians and Normalized MAD (nMAD = 1.4826*MAD) of each group are reported for each day of measurement.

Tumor volume changes from baseline were calculated for each animal and each day by subtracting the tumor volume on the day of first treatment (day 0) from the tumor volume on the specified observation day.

All statistical analyses were performed using SAS version 9.2 software. A probability of less than 5% ($p<0.05$) was considered as significant.

## 2: Results

Amcenestrant at 10 mg/kg BID, everolimus 10 mg/kg BIW and the combination of amcenestrant and everolimus at the doses and regime for 28 days were tolerated, and no drug-related body weight loss/animal death was observed in the study. But we observed E2 supplement-related significant body weight loss or death, which was excluded from data statistical analysis.

Amcenestrant at a dose of 10 mg/kg BID for 28 days had statistically significant anti-tumor efficacy with ΔT/ΔC value of -13% ($p <0.0001$) on day 28. Everolimus at a dose of 10 mg/kg BIW for 28 days induced statistically significant anti-tumor efficacy with ΔT/ΔC value of -17% ($p <0.0001$) on day 28. When amcenestrant at 10 mg/kg combined with everolimus 10 mg/kg with the same dose regime as BID for amcenestrant and BIW for everolimus, the combination treatment demonstrated statistically significant anti-tumor efficacy (tumor regression) with ΔT/ΔC value of - 31% ($p < 0.0001$) on day 28. The statistical analysis indicated that the combination effect was significantly different when compared to either amcenestrant alone or everolimus alone on day 28 ($p < 0.0001$).

Detailed results are shown in Tables 1 to 3 below, as well as in Figures 1 and 2.

Brief description of the drawings:

- Figure 1: Antitumor activity of amcenestrant combined with everolimus against orthotopic human breast cancer cell line MCF7 xenograft in NOD/SCID mice: tumor volume evolution. The curves represent medians + or - MAD (Median Absolute Deviation) at each day for each group.

- Figure 2: Antitumor activity of amcenestrant combined with everolimus against orthotopic human breast cancer cell line MCF7 xenograft in NOD/SCID mice: tumor volume changes from baseline on day 28. Points represent individual tumor volume changes from baseline on day 28, bars correspond to medians.

From this experiment, it was concluded that amcenestrant at 10 mg/kg twice a day combined with the mTOR inhibitor everolimus at 10 mg/kg twice a week in MCF7 human breast cancer cell line xenograft model in NOD/SCID mice induced significant anti-tumor efficacy that was superior to single agents alone, and induced tumor growth inhibition and tumor regression.

**Table 1:** Efficacy of amcenestrant (SAR439859) combined with everolimus against orthotopic MCF7 human breast cancer xenograft in NOD/SCID mice. PO: per os

| Agent | Route/ Dosage (in mL/kg per injection) | Dosage in mg/kg per injection | Schedule in days (total of 28 days) | *Unscheduled death (Day of death) | ΔT/ΔC in % at day 28 | Median % of regressions on day 28 | Regressions | | p-value on day 28 | Biological Interpretation |
|---|---|---|---|---|---|---|---|---|---|---|
| | | | | | | | Partial | Complete | | |
| Vehicle | PO, BID (10) | - | - | 0/7 | 100 | - | 0/7 | 0/7 | - | - |
| SAR439859 | PO, BID (10) | 10 | 0 to 28 | 0/9 | -13 | -13 | 8/9 | 0/9 | $p < 0.0001$ | Very active |
| Everolimus | PO, BIW (10) | 10 | 0 to 28 | 0/6 | -17 | -17 | 5/6 | 0/6 | $p < 0.0001$ | Very active |
| SAR439859 + Everolimus | PO, BID (10)<br>PO, BIW (10) | 10 + 10 | 0 to 28 | 0/7 | -31 | -31 | 6/7 | 1/7 | $p < 0.0001$ | Very active |

**Table 2:** Efficacy of amcenestrant (SAR439859) combined with everolimus against orthotopic human breast cancer cell line MCF7 xenograft model in NOD/SCID mice. Comparison of each group to the control group at each day.

| Tumor volume changes from baseline mm$^3$ : Median (nMAD), n and p-value | | | | | | | | | |
|---|---|---|---|---|---|---|---|---|---|
| **Treatment Group** | **Global** | **Day 4** | **Day 7** | **Day 11** | **Day 14** | **Day 18** | **Day 21** | **Day 25** | **Day 28** |
| Control | - | 54.0 (29.65) n=10 | 91.0 (19.27) n=9 | 170.0 (16.31) n=9 | 195.0 (26.69) n=9 | 216.0 (35.58) n=7 | 258.0 (29.65) n=7 | 292.0 (40.03) n=7 | 312.0 (47.44) n=7 |
| Everolimus 10 mg/kg BIW | - | 28.5 (12.60) n=10 | 48.0 (14.83) n=9 | 65.0 (26.69) n=9 | 81.5 (87.47) n=8 | 10.0 (60.79) n=7 | -48.0 (13.34) n=7 | -47.0 (51.89) n=7 | -53.0 (20.76) n=6 |
| | **<.0001** | 0.0756 | **0.0026** | **0.0035** | **<.0001** | **<.0001** | **<.0001** | **<.0001** | **<.0001** |
| Amcenestrant 10 mg/kg BID | - | 22.0 (14.83) n=10 | 32.5 (14.83) n=10 | 60.0 (10.38) n=10 | 76.5 (37.81) n=10 | 40.0 (23.72) n=9 | -2.0 (19.27) n=9 | -38.0 (16.31) n=9 | -42.0 (16.31) n=9 |
| | **<.0001** | **0.0238** | **<.0001** | **0.0008** | **<.0001** | **<.0001** | **<.0001** | **<.0001** | **<.0001** |
| Everolimus 10 mg/kg BIW + amcenestrant 10 mg/kg BID | - | 10.0 (2.22) n=10 | 18.5 (5.93) n=10 | -53.5 (9.64) n=8 | -70.0 (16.31) n=8 | -86.5 (11.86) n=8 | -92.5 (8.15) n=8 | -94.0 (2.97) n=7 | -97.0 (4.45) n=7 |
| | **<.0001** | **<.0001** | **<.0001** | **<.0001** | **<.0001** | **<.0001** | **<.0001** | **<.0001** | **<.0001** |
| # p-values obtained with a contrast analysis versus control at each day with Bonferroni-Holm adjustment for multiplicity after a two-way Anova-Type on tumor volume changes from baseline. MAD= Median Absolute Deviation; nMAD= normalized MAD ; nMAD= 1.4826*MAD | | | | | | | | | |

**Table 3:** Efficacy of amcenestrant (SAR439859) combined with everolimus against orthotopic human breast cancer cell line MCF7 xenograft model in NOD/SCID mice. Comparison of amcenestrant 10 mg/kg and everolimus 10 mg/kg as single agents *versus* the combination at each day.

| Tumor volume changes from baseline mm$^3$ : Median (nMAD), n and p-value | | | | | | | | | |
|---|---|---|---|---|---|---|---|---|---|
| **Treatment Group** | **Global** | **Day 4** | **Day 7** | **Day 11** | **Day 14** | **Day 18** | **Day 21** | **Day 25** | **Day 28** |
| Everolimus 10 mg/kg BIW + amcenestrant 10 mg/kg BID | - | 10.0 (2.22) n=10 | 18.5 (5.93) n=10 | -53.5 (9.64) n=8 | -70.0 (16.31) n=8 | -86.5 (11.86) n=8 | -92.5 (8.15) n=8 | -94.0 (2.97) n=7 | -97.0 (4.45) n=7 |
| Amcenestrant 10 mg/kg BID | - <br> **<.0001** | 22.0 (14.83) n=10 **0.0339** | 32.5 (14.83) n=10 **0.0282** | 60.0 (10.38) n=10 **<.0001** | 76.5 (37.81) n=10 **<.0001** | 40.0 (23.72) n=9 **<.0001** | -2.0 (19.27) n=9 **<.0001** | -38.0 (16.31) n=9 **0.0003** | -42.0 (16.31) n=9 **0.0007** |
| Everolimus 10 mg/kg BIW | - <br> **<.0001** | 28.5 (12.60) n=10 **0.0085** | 48.0 (14.83) n=9 **0.0014** | 65.0 (26.69) n=9 **<.0001** | 81.5 (87.47) n=8 **<.0001** | 10.0 (60.79) n=7 **<.0001** | -48.0 (13.34) n=7 **<.0001** | -47.0 (51.89) n=7 **<.0001** | -53.0 (20.76) n=6 **<.0001** |
| # p-values obtained with a contrast analysis to compare the combinations of everolimus and amcenestrant versus each single agent at the dose involved in the combination at each day with Bonferroni-Holm adjustment for multiplicity after a two-way Anova-Type on tumor volume changes from baseline. MAD= Median Absolute Deviation; nMAD= normalized MAD ; nMAD= 1.4826*MAD | | | | | | | | | |

## Claims

1. A combination comprising amcenestrant, or a pharmaceutically acceptable salt thereof, and everolimus, for use in the treatment of breast cancer.

2. The combination for use according to claim 1, wherein amcenestrant, or a pharmaceutically acceptable salt thereof, and everolimus are administered separately, simultaneously or spaced out over a period of time.

3. A pharmaceutical composition comprising amcenestrant, or a pharmaceutically acceptable salt thereof, and everolimus, and at least one pharmaceutically acceptable excipient, for use in the treatment of breast cancer.

4. Amcenestrant or a pharmaceutically acceptable salt thereof for use in the treatment of breast cancer by co-administration with everolimus.

5. The compound for use in the treatment of breast cancer according to claim 4, which is administered separately, simultaneously, or spaced out over time, with everolimus.

6. Everolimus for use in the treatment of breast cancer by co-administration with amcenestrant or a pharmaceutically acceptable salt thereof.

7. Everolimus for use in the treatment of breast cancer according to claim 6, which is administered separately, simultaneously, or spaced out over time, with amcenestrant or a pharmaceutically acceptable salt thereof.

8. A pharmaceutical kit comprising:

   (i) a first pharmaceutical composition comprising amcenestrant, or a pharmaceutically acceptable salt thereof, and at least one pharmaceutically acceptable excipient;
   (ii) a second pharmaceutical composition comprising everolimus, and at least one pharmaceutically acceptable excipient;

   wherein the first pharmaceutical composition and the second pharmaceutical composition are in separate compartments and are intended to be independently administered, each administration with regards to the other one being simultaneous or spaced out over time, for use in the treatment of breast cancer.

## Patentansprüche

1. Kombination, umfassend Amcenestrant oder ein pharmazeutisch akzeptables Salz davon und Everolimus, zur Verwendung bei der Behandlung von Brustkrebs.

2. Kombination zur Verwendung gemäß Anspruch 1, wobei Amcenestrant oder ein pharmazeutisch akzeptables Salz davon und Everolimus separat, gleichzeitig oder zeitlich beabstandet verabreicht werden.

3. Pharmazeutische Zusammensetzung, umfassend Amcenestrant oder ein pharmazeutisch akzeptables Salz davon und Everolimus und mindestens einen pharmazeutisch akzeptablen Hilfsstoff, zur Verwendung bei der Behandlung von Brustkrebs.

4. Amcenestrant oder ein pharmazeutisch akzeptables Salz davon zur Verwendung bei der Behandlung von Brustkrebs durch Co-verabreichung mit Everolimus.

5. Die Verbindung zur Verwendung bei der Behandlung von Brustkrebs gemäß Anspruch 4, die separat, gleichzeitig oder zeitlich beabstandet mit Everolimus verabreicht wird.

6. Everolimus zur Verwendung bei der Behandlung von Brustkrebs durch Co-verabreichung mit Amcenestrant oder einem pharmazeutisch akzeptablen Salz davon.

7. Everolimus zur Verwendung bei der Behandlung von Brustkrebs gemäß Anspruch 6, das separat, gleichzeitig oder zeitlich beabstandet mit Amcenestrant oder einem pharmazeutisch akzeptablen Salz davon verabreicht wird.

**8.** Pharmazeutisches Kit, umfassend:

(i) eine erste pharmazeutische Zusammensetzung, umfassend Amcenestrant oder ein pharmazeutisch akzeptables Salz davon und mindestens einen pharmazeutisch akzeptablen Hilfsstoff;
(ii) eine zweite pharmazeutische Zusammensetzung, umfassend Everolimus und mindestens einen pharmazeutisch akzeptablen Hilfsstoff;

wobei die erste pharmazeutische Zusammensetzung und die zweite pharmazeutische Zusammensetzung in separaten Kompartimenten sind und zur unabhängigen Verabreichung vorgesehen sind, wobei jede Verabreichung bezüglich der anderen gleichzeitig oder zeitlich beabstandet erfolgt, zur Verwendung bei der Behandlung von Brustkrebs.

**Revendications**

**1.** Combinaison comprenant de l'amcénestrant, ou un sel pharmaceutiquement acceptable de celui-ci, et de l'évérolimus, pour utilisation dans le traitement du cancer du sein.

**2.** Combinaison pour utilisation selon la revendication 1, dans laquelle l'amcénestrant, ou un sel pharmaceutiquement acceptable de celui-ci, et l'évérolimus sont administrés séparément, simultanément ou espacés sur une période de temps.

**3.** Composition pharmaceutique comprenant de l'amcénestrant, ou un sel pharmaceutiquement acceptable de celui-ci, et de l'évérolimus, et au moins un excipient pharmaceutiquement acceptable, pour utilisation dans le traitement du cancer du sein.

**4.** Amcénestrant ou un sel pharmaceutiquement acceptable de celui-ci, pour utilisation dans le traitement du cancer du sein par co-administration avec de l'évérolimus.

**5.** Le composé pour utilisation dans le traitement du cancer du sein selon la revendication 4, qui est administré séparément, simultanément ou espacé dans le temps, avec de l'évérolimus.

**6.** Évérolimus pour utilisation dans le traitement du cancer du sein par co-administration avec de l'amcénestrant ou un sel pharmaceutiquement acceptable de celui-ci.

**7.** Évérolimus pour utilisation dans le traitement du cancer du sein selon la revendication 6, qui est administré séparément, simultanément ou espacé dans le temps, avec de l'amcénestrant ou un sel pharmaceutiquement acceptable de celui-ci.

**8.** Kit pharmaceutique comprenant :

(i) une première composition pharmaceutique comprenant de l'amcénestrant ou un sel pharmaceutiquement acceptable de celui-ci, et au moins un excipient pharmaceutiquement acceptable ;
(ii) une seconde composition pharmaceutique comprenant de l'évérolimus, et au moins un excipient pharmaceutiquement acceptable ;

dans lequel la première composition pharmaceutique et la seconde composition pharmaceutique sont dans des compartiments distincts et sont destinées à être administrées indépendamment, chaque administration par rapport à l'autre étant simultanée ou espacée dans le temps, pour utilisation dans le traitement du cancer du sein.

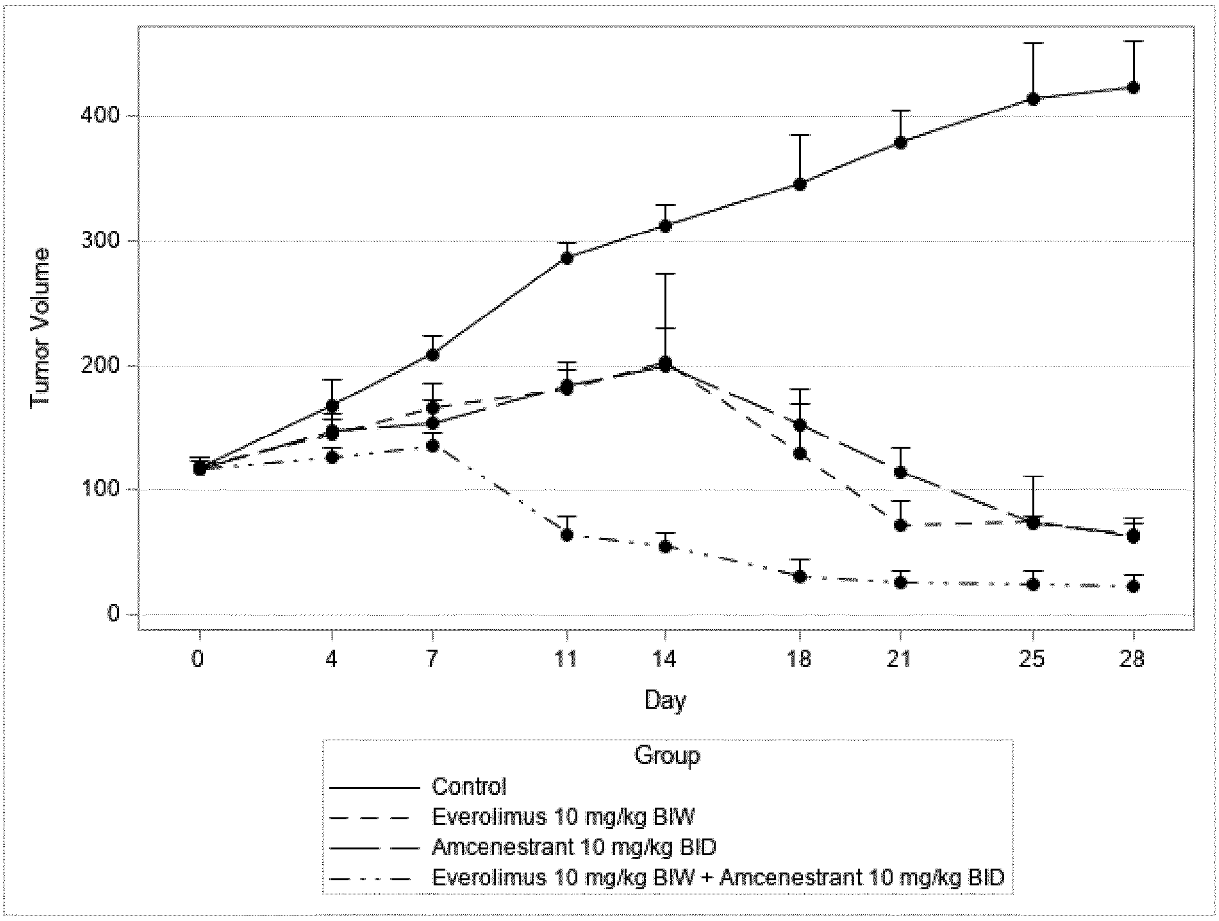
400
300
200
100
0
Tumor Volume
0
4
7
11
14
18
21
25
28
Day
Group
Control
Everolimus 10 mg/kg BIW
Amcenestrant 10 mg/kg BID
Everolimus 10 mg/kg BIW + Amcenestrant 10 mg/kg BID

**Figure 1**

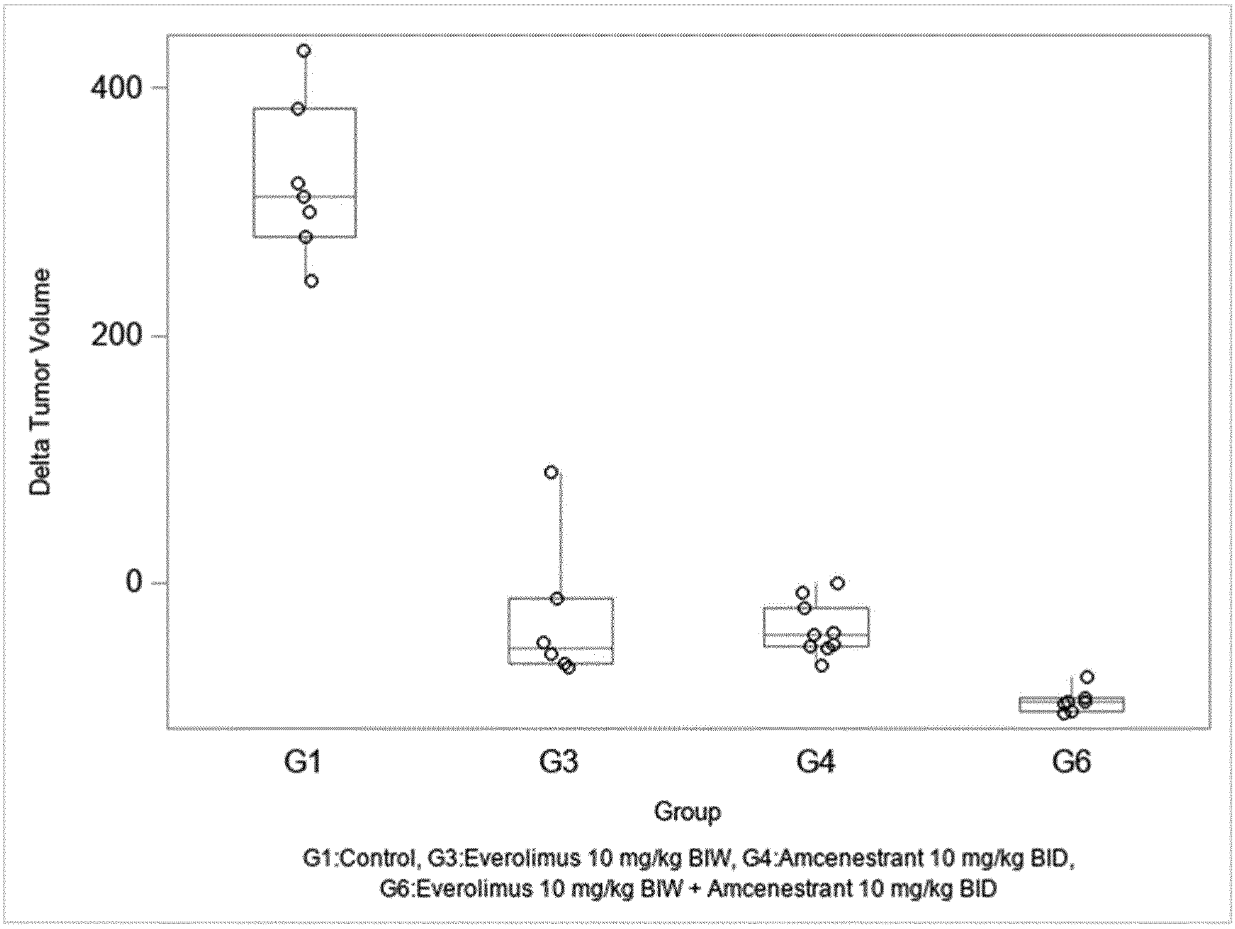
400
200
0
Delta Tumor Volume
G1
G3
G4
G6
Group
G1:Control, G3:Everolimus 10 mg/kg BIW, G4:Amcenestrant 10 mg/kg BID,
G6:Everolimus 10 mg/kg BIW + Amcenestrant 10 mg/kg BID

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017140669 A